# EUROPÄISCHE PATENTSCHRIFT

(11) **EP 2 500 412 B1**
(45) Veröffentlichungstag und Bekanntmachung des Hinweises auf die Patenterteilung: **29.10.2014**
(21) Anmeldenummer: 11002143.3
(22) Anmeldetag: 15.03.2011
(51) Int. Cl.: C12N 5/00

(54) **Verfahren zur Produktion von Fischzellen mit erhöhtem Gehalt an hochgradig ungesättigten Fettsäuren und Verwendung dieser Fischzellen zur Gewinnung von fischspezifischen Produkten**
Method for producing fish cells with increased high quality unsaturated fatty acids and use of these fish cells to produce fish-specific products
Procédé de production de cellules de poisson ayant une teneur plus élevée en acides gras hautement insaturés et utilisation de ces cellules de poisson pour la production de produits spécifiques aux poissons

(43) Veröffentlichungstag der Anmeldung: 19.09.2012
(73) Patentinhaber: Fraunhofer-Gesellschaft zur Förderung der angewandten Forschung, 80686 München (DE)
(72) Erfinder: Kruse, Charli, 23923 Herrnburg (DE); Gebert, Marina, 22763 Hamburg (DE); Lüllwitz, Lars, 23556 Lübeck (DE)
(74) Vertreter: Katzameyer, Michael

(56) Entgegenhaltungen:
- DE-A1-102007 029 699
- RUYTER B ET AL: "Influence of temperature and high dietary linoleic acid content on esterification, elongation, and desaturation of PUFA in Atlantic salmon hepatocytes.", LIPIDS AUG 2003 LNKD- PUBMED:14577662, Bd. 38, Nr. 8, August 2003 (2003-08), Seiten 833-840, XP9149917, ISSN: 0024-4201
- MILLER MATTHEW R ET AL: "n-3 Oil sources for use in aquaculture--alternatives to the unsustainable harvest of wild fish.", NUTRITION RESEARCH REVIEWS DEC 2008 LNKD- PUBMED:19087364, Bd. 21, Nr. 2, Dezember 2008 (2008-12), Seiten 85-96, XP9149921, ISSN: 1475-2700
- BUTLER M ET AL: "Unsaturated fatty acids enhance cell yields and perturb the energy metabolism of an antibody-secreting hybridoma.", THE BIOCHEMICAL JOURNAL 1 MAR 1997 LNKD- PUBMED:9065785, Bd. 322 ( Pt 2), 1. März 1997 (1997-03-01) , Seiten 615-623, XP9149918, ISSN: 0264-6021
- TOCHER D R ET AL: "Effects of n-3 and n-6 polyunsaturated fatty acids on the growth of fish cells in culture.", BIOCHEMICAL SOCIETY TRANSACTIONS OCT 1990 LNKD- PUBMED:2083730, Bd. 18, Nr. 5, Oktober 1990 (1990-10), Seiten 915-916, XP9149925, ISSN: 0300-5127
- LEAVER MICHAEL J ET AL: "Functional genomics reveals increases in cholesterol biosynthetic genes and highly unsaturated fatty acid biosynthesis after dietary substitution of fish oil with vegetable oils in Atlantic salmon (Salmo salar)", BMC GENOMICS, Bd. 9, Juni 2008 (2008-06), XP21033038, ISSN: 1471-2164

## Beschreibung

### Hintergrund

Die Erfindung betrifft ein Verfahren zur Produktion von Fischzellen mit erhöhtem Gehalt an hochgradig ungesättigten Fettsäuren und die Verwendung dieser Fischzellen zur Gewinnung von fischspezifischen Produkten wie z.B. Fischmehl, Fischproteinen und Fischölen, sowie dafür geeignete Fischzellen und Fischzellkulturen.

Seit Mitte der 90iger Jahre des letzten Jahrtausends stagnieren die Wildfischfänge bei einer Menge von etwa 90 Millionen Tonnen pro Jahr. Fast zwei Drittel davon erreichen direkt den Konsumenten als Speisefisch. Über ein Drittel und damit mehr als 30 Millionen Tonnen der Wildfischfänge finden keine Verwendung als Speisefisch. Diese 30 Millionen Tonnen werden jedes Jahr unter anderem für Tiernahrung in Form von Fischmehl und Fischöl, als Dosenfutter oder Aquakulturfutter zur Züchtung von Speisefischen weiterverarbeitet. Das Aquakulturfutter setzt sich aus vielen Komponenten zusammen. Zwei dieser Komponenten sind eben genanntes Fischmehl und Fischöl, deren Zugabe je nach Fischart variiert. Der Anteil an Fischmehl liegt zwischen 5 und 50 % und bei Fischöl zwischen 0 und 16%. Mit den steigenden Bevölkerungszahlen geht auch ein erhöhter Bedarf an Speisefisch einher. Dieser kann schon seit vielen Jahren nur durch zusätzlichen Fisch aus Aquakultur gedeckt werden. Die rasant steigenden Produktionsmengen der Aquakulturbetriebe in den letzten 10 Jahren und der damit erhöhte Verbrauch an Fischmehl und Fischöl reduzieren die ohnehin schon raren Mehl- und Ölressourcen. Der Marktpreis für Fischmehl und Fischöl wird sich deshalb vermutlich schon in den kommenden Jahren stark erhöhen.

Die bisherigen Quellen für Fischmehl sind neben Wildfischfängen und deren Beifänge, die kein industrielles Interesse decken, auch Fischabfälle aus der fischverarbeitenden Industrie. Die Quellen für Fischöl entsprechen denen des Fischmehls. Spezielle Omega-3-Fettsäuren können auch aus Algen gewonnen werden. Aus den oben genannten Gründen besteht schon seit einiger Zeit die Notwendigkeit die Fischmehl- und Fischölanteile in den speziellen Futtermischungen durch Beimischung von Pflanzenölen (Rapsöl, Sojaöl und Leinöl) und Fleischzusätzen aus der Viehhaltung zu ersetzen. Diese untypischen Futterzusammensetzungen verändern das Verhältnis von Fischprotein zu Fischfett im Zuchtfisch. Insbesondere der wünschenswerte Anteil an hochgradig ungesättigten Omega-3- und Omega-6-Fettsäuren wird reduziert, wenn Fischöl durch Pflanzenöle ersetzt wird. Dadurch werden die gesundheitsförderlichen Effekte einer fischreichen Ernährung verringert.

Zur Gewinnung von Fischproteinen, Fischölen und anderen Fischprodukten wurde auch die Verwendung von in-vitro-Fischzellkulturen bereits grundsätzlich in Betracht gezogen.

DE 10 207 029 699.3 und EP 08 707 810.1 offenbaren ein Verfahren zur Herstellung von proliferierenden Fischzellen mit Stammzelleigenschaften aus der Kopfniere von Fischen und erwähnen deren mögliche Verwendung zur Produktion von fischspezifischer Biomasse, einschließlich Fischproteinen, Fischölen und Omega-Fettsäuren. Diese Druckschriften enthalten jedoch keinerlei Hinweise hinsichtlich einer möglichen Optimierung des Gehalts an hochgradig ungesättigten Fettsäuren in diesen Fischzellen bzw. der daraus erhaltenen Biomasse.

Ruyter et al. (in LIPIDS, Bd. 38, Nr. 8, (2003), 833-840) beschreiben eine primäre Hepatozytenzellkultur mit erhöhtem Gehalt an ungesättigten Fettsäuren, erhalten aus Fischen, die bei tiefen Temperaturen kultiviert wurden.

Der Erfindung liegt daher die Aufgabe zu Grunde, neue verbesserte Mittel bereitzustellen, mit denen der geschilderte dringende Bedarf an Fischmehl, Fischproteinen und Fischöl, insbesondere mit einem hohen Gehalt/Anteil an hochgradig ungesättigten Omega-3- und Omega-6-Fettsäuren, auf einfache und kostengünstige Weise effizient gedeckt werden kann.

Diese Aufgabe wird erfindungsgemäß gelöst durch die Bereitstellung der Verfahren nach Anspruch 1 und 12 sowie der Fischzellkultur nach Anspruch 9. Weitere Aspekte und/oder bevorzugte Ausführungsformen der Erfindung sind Gegenstand der weiteren Ansprüche.

### Beschreibung derErfindung

Die Erfindung betrifft ein Verfahren zur Produktion von Fischzellen mit einem erhöhten Gehalt an hochgradig ungesättigten Fettsäuren mit den Merkmalen nach Anspruch 1. Das erfindungsgemäße Verfahren umfasst das Kultivieren und Vermehren von proliferierenden Fischzellen bei einer Temperatur von 1-35 °C, insbesondere 4-30 °C oder 10-30 °C, in einem Zellkulturmedium und ist u.a. dadurch gekennzeichnet, dass das Kultivieren und Vermehren der proliferierenden Fischzellen unter den folgenden Bedingungen geschieht:
a) das Zellkulturmedium enthält einen Zusatz von mindestens einer kurzkettigen, mehrfach ungesättigten Fettsäure in einer Konzentration von 1-500 µM;
b) das Kultivieren der Fischzellen erfolgt bei mindestens 2, vorzugsweise mindestens 3, verschiedenen Temperaturen.

Der Begriff "hochgradig ungesättigte Fettsäuren (HUFAS, "highly unsaturated fatty acids"), wie hier verwendet, bedeutet Fettsäuren, die eine Kohlenstoffkettenlänge von 20 oder mehr C-Atomen und drei oder mehr Kohlenstoff-Doppelbindungen aufweisen.

Bevorzugte Vertreter dieser hochgradig ungesättigten Fettsäuren sind Eicosapentaensäure (EPA, C20.5n3) und Docosahexaensäure (DHA, C22:6n3).

Der Begriff "kurzkettige, mehrfach ungesättigte Fettsäure", wie hier verwendet, bedeutet eine Fettsäure mit einer Kettenlänge von weniger als 20 C-Atomen, vorzugsweise bis 18 C-Atomen, die zwei oder mehr Kohlenstoff-Doppelbindungen aufweist. Vorzugsweise ist die im erfindungsgemäßen Verfahren verwendete Fettsäure eine Fettsäure, die bekanntermaßen bei der in vivo-Synthese von langkettigen mehrfach ungesättigten Fettsäuren (Säuger oder Fisch) eine Rolle spielt.

Die im erfindungsgemäßen Verfahren verwendete kurzkettige ungesättigte Fettsäure ist aus der Gruppe aus Alpha-Linolensäure (ALA, 18:3n3), Palmitolensäure (16:1n7), Olensäure (18:1n9) und Linolsäure (18:2n6) ausgewählt.

Bei Durchführung des erfindungsgemäßen Verfahrens unter Bedingung a) enthält das Zellkulturmedium die kurzkettige ungesättigte Fettsäure in einer Konzentration von 1 bis 500 µM, vorzugsweise 5 bis 500 µM oder 10 bis 300 µM, bevorzugter von 20 bis 200 µM, z.B. 30 bis 150 µM. Bereits eine Konzentration von 1 µM liegt typischerweise deutlich, etwa um einen Faktor 5-10, über dem Gehalt solcher Fettsäuren in serumhaltigem Medium ohne speziellen Zusatz einer Fettsäure.

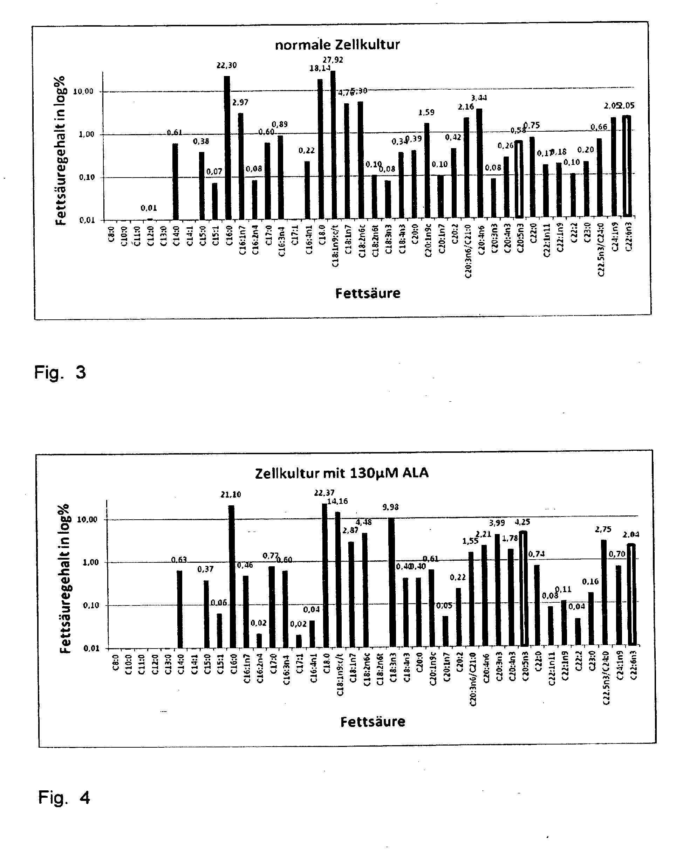

Die Beispiele demonstrieren, dass der Zusatz von 130 µM ALA zum Zellkulturmedium zu einer Erhöhung des Gehalts/Anteils an ALA [dunkler Balken; 18:3n3] in den *in vitro* kultivierten Fischzellen um den Faktor 125 (vgl. Fig. 3 (normale Zellkultur; [0,08%]) und Fig. 4 (Zellkultur mit 130 µM ALA; [9,98%]) führte. Neben der Zunahme der ALA konnte auch gezeigt werden, dass die Gesamtheit der ungesättigten Fettsäuren, die länger als ALA sind, also rechts vom ALA-Balken auftauchen, um ca. 6,5% zunahmen (vgl. normale Zellkultur [Summe = 15,52%] und Zellkultur mit 130 µM ALA [Summe = 22,08%]). Die Fischzellen in dieser Fischzellkultur sind also in der Lage, den Zusatz an kurzkettigen Fettsäuren in langkettige, höherwertige Fettsäuren zu verstoffwechseln.

Dies gilt vor allem auch für die Fettsäuren Eicosapentaensaure (EPA, C20:5n3) und Docosahexaensaure (DHA, C22:6n3), die von besonderem gesundheitlichen und ernährungsphysiologischen Interesse sind.

Tocher und Dick berichteten bereits über den Zusatz von ALA zu einer Lachszellkultur (Fish Physiology and Biochemistry, Bd. 8, Nr. 4, 311-319 (1990)), verwendeten jedoch eine andere Methode zu Einbringung der Fettsäure in das Medium und beobachteten keine so deutlichen Erhöhungen des EPA- und DHA-Gehalts wie mit dem erfindungsgemäßen Verfahren erreicht.

Untersuchungen der Erfinder haben ferner ergeben, dass eine Kultur unter Bedingung b), d.h. Kultivierung bei mindestens 2, vorzugsweise mindestens 3, verschiedenen Temperaturen, ebenfalls zu einer Erhöhung des Anteils an langkettigen ungesättigten Fettsäuren wie EPA und DHA führt.

Die Fischzellen werden dabei zuerst bei einer hohen Temperatur im Bereich von 20-30 °C, vorzugsweise 22-28 °C, und dann bei einer niedrigen Temperatur unterhalb von 20 °C kultiviert.

Die hohe Temperatur entspricht etwa dem Wachstumsoptimum der Fischzellen und die niedrige Temperatur liegt mindestens 5-15 °C, vorzugsweise mindestens 8-12 °C, z.B. 10-20 °C oder 10-30 °C, tiefer.

Besonders bevorzugt wird das Verfahren dabei so durchgeführt, dass zwischen der Kultivierung bei der hohen Temperatur und der Kultivierung bei der niedrigen Temperatur noch eine Kultivierung der Fischzellen bei mindestens einer dazwischen liegenden Temperatur erfolgt. Diese Adaptionsphase verbessert die Ausbeute an Fischzellen deutlich.

Typischerweise werden die Fischzellen für einen Zeitraum von 1-3 Tagen, vorzugsweise 40-55 h, bei der hohen Temperatur, für einen Zeitraum von 1-3 Tagen, vorzugsweise 40-55 h, bei der mittleren Temperatur, und für einen Zeitraum von 2-4 Tagen, vorzugsweise ca. 60-80 h, bei der niedrigen Temperatur kultiviert.

Die optimalen Kultivierungsbedingungen, insbesondere hinsichtlich Temperatur und Zeitdauer der verschiedenen Kultivierungsphasen, können in Abhängigkeit von der jeweiligen Spezies und Zelllinie der verwendeten Fischzellen variieren, sind jedoch unschwer vom Fachmann durch Routineexperimente zu ermitteln.

Im Beispielsabschnitt wird ein auf DMEM basierendes Zellkulturmedium beschrieben, mit dem sehr gute Ergebnisse erzielt wurden. Statt diesem Medium könnte jedoch auch ohne Weiteres ein anderes übliches Zellkulturmedium, vorzugsweise ein für die Kultivierung von Fischzellen bekanntes Medium, verwendet werden. Insbesondere kann statt FKS (fötalem Kälberserum) auch Karpfenserum oder ein anderer äquivalenter Zusatz verwendet werden.

Die erfindungsgemäß zur Kultivierung verwendeten proliferierenden Fischzellen sind nicht besonders beschränkt. Es können sowohl bekannte Fischzelllinien des Standes der Technik verwendet (z.B. RTG-2 (Tocher et al., Comp. Biochem. Physiol. 94B:367-374 (1989), RTL-W1) als auch neue Zelllinien aus Fischgewebe etabliert werden. Das Ausgangsgewebe für eine Fischzell-Primärkultur kann z.B. das Gesamtgewebe einer Fischlarve oder ein bestimmtes Organ eines adultes Zielfisches sein.

Als adultes Ausgangsgewebe kann beispielsweise Gewebe aus einer Niere, insbesondere der Kopfniere, der Leber, dem Pankreas bzw. den Pylorusanhängen, Darm, Herz, Gehirn, den Gonaden, dem Fettgewebe, der Haut, oder Muskelgewebe verwendet werden.

In einer bevorzugten Ausführungsform wird das Gesamtgewebe einer Fischlarve verwendet. Der Begriff "Fischlarven", wie hier verwendet, umfasst insbesondere Fischeier oder Fischlarven ab dem Augenpunktstadium (Ausbildung von Augenpunkten) bis zum Ende des Dottersackstadiums (Ernährung vom Dottersack).

Als Quelle für die erfindungsgemäß verwendeten Fischlarven oder Fischgewebe sind grundsätzlich beliebige Fischspezies geeignet, vorzugsweise solche der Osteichthyes, inbesondere Spezies der Teleostei. Spezielle Beispiele hierfür sind Spezies der Heringsfische (Clupeoidei), Lachsartigen (Salmonoidei), z.B. Lachse, Forellen, insbesondere Bachforelle, Regenbogenforelle, Saiblinge, Huchen, Felchen, Renken, Äschen, Stinte, der Karpfenfische (Cyprinidae), Aale (Anguillidae), Barsche (Percidae), Dorsche (Gadidae), Welse (Siluroidae), Plattfische (Pleuronectiformes), Hornhechte (Beloniformes), Störe (Acipenseriformes) etc.

Allgemein bevorzugt sind Fischspezies, die bereits natürlicherweise einen hohen Gehalt an hochgradig ungesättigten Fettsäuren aufweisen.

Einige spezielle, nicht-beschränkende Beispiele für geeignete Spezies sind Stör, Hering, Forelle, Lachs, Aal, Karpfen, Makrele, Heilbutt, Sardine.

Besonders gute Ergebnisse werden mit einer Kombination der beiden oben beschriebenen Kultivierungsbedingungen a) (Zusatz einer relativ kurzkettigen mehrfach ungesättigten Fettsäure wie ALA) und b) (zeitweise Kultivierung bei tiefer Temperatur: "Kaltstellung") erzielt.

Mit dem erfindungsgemäßen Verfahren sind Fischzellen erhältlich, welche einen Gehalt an hochgradig ungesättigten Fettsäuren, insbesondere Eicosapentaensäure (EPA, C20.5n3) und/oder Docosahexaensäure (DHA, C22:6n3), aufweisen, der gegenüber dem Gehalt in Fischzellen der gleichen Fischzelllinie, die bei einer Kultivierung im gleichen Zellkulturmedium ohne einen Zusatz von mindestens einer kurzkettigen, mehrfach ungesättigten Fettsäure und bei einer Temperatur, die dem Wachstumsoptimum dieser Fischzellen entspricht, erhalten werden, deutlich erhöht ist. Vorzugsweise ist der Gehalt an EPA + DHA um einen Faktor von mindestens 2, bevorzugter mindestens 2,5 oder 3, z.B. mindestens 5, erhöht.

Die durchgeführten Experimente belegen, dass solche Werte mit dem erfindungsgemäßen Verfahren problemlos erreicht werden können. Bei normaler Zellkultur (Referenzbeispiel) sind 2,6 % der Zellmasse EPA und DHA (HUFAs insgesamt 5 %), durch Zugabe von 130 µM ALA erhöht sich die Summe von EPA und DHA auf 6,3 %, also um einen Faktor von 2,4, durch Kaltstellen allein ergab sich ein Faktor von 2,85, durch Zugabe von 65 µM ALA plus Kaltstellen wurde ein Faktor von 5,96 erreicht.

Ein weiterer verwandter Aspekt der Erfindung betrifft eine Fischzellkultur mit den Merkmalen von Anspruch 9, umfassend proliferierende Fischzellen in einem Zellkulturmedium, das einen Zusatz von mindestens einer kurzkettigen mehrfach ungesättigten Fettsäure enthält, und die vorzugsweise eine Temperatur im Bereich von 1-20 °C, insbesondere 4-20°C, aufweist.

Gegenstand der vorliegenden Erfindung ist auch die Verwendung der Fischzellkultur zur Gewinnung von fischspezifischer Biomasse und fischspezifischen Produkten wie Fischmehl, Fischproteinen, Fischölen oder Fettsäuren, insbesondere hochgradig ungesättigten Fettsäuren. Diese Fischzellen bzw. fischspezifischen Produkte können z.B. als Quelle von langkettigen hochwertigsten Fettsäuren, Vitaminen und Enzymen in der Ernährungsindustrie (Nahrungsergänzungsmittel, diätetische Lebensmittel), als Tierfutterzusatz, in der Medizin oder Pharmazie eingesetzt werden. Die Fettsäuren können dabei als Gesamtextrakt oder als isolierte Produkte Verwendung finden.

Auch die Aminosäurezusammensetzung der Fischzellen ist durch das Vorkommen von sowohl nicht-essentiellen als auch essentiellen Aminosäuren sehr vorteilhaft und ermöglicht die Gewinnung hochwertiger Fischproteine.

Mit der in vitro-Fischzellkultur und Expansion der Zellen können Fischmehl und andere fischspezifische Produkte wie beispielsweise Fischöle oder ungesättigte Fettsäuren großtechnisch in z.B. Hochdurchsatz-Reaktoren hergestellt werden.

Ein noch weiterer Gegenstand der Erfindung ist ein Verfahren zur Gewinnung von fischspezifischer Biomasse und fischspezifischen Produkten wie Fischmehl, Fischproteinen, Fischölen und/oder Fettsäuren aus Fischzellkulturen, umfassend
a) Kultivieren und Vermehren von proliferierenden Fischzellen in einem Zellkulturmedium gemäß dem Verfahren nach einem der Ansprüche 1-8,
b) Ernten der Fischzellen, und
c) Gefriertrocknen und/oder Zerkleinern und/oder Aufschließen der geernteten Fischzellen.

### FIGURENBESCHREIBUNG

**Fig. 1** zeigt die relative Aminosäurezusammensetzung der Fischzellen bei Kultivierung gemäß Referenzbeispiel 1 ("normale Zellkultur") (jeweils linker Balken) im Vergleich zu normalem Fischmuskelfleisch (jeweils rechter Balken) .
**Fig. 2** zeigt die prozentuale Fettsäurezusammensetzung des DMEM-Nährmediums ohne ALA-Zusatz.
**Fig. 3** zeigt den prozentualen Gehalt verschiedener Fettsäuren in den Fischzellen, die bei Kultivierung ohne ALA und ohne anschließendem Temperaturwechsel gemäß Referenzbeispiel 1 ("normale Zellkultur") erhalten wurden.
**Fig. 4** zeigt den Gehalt verschiedener Fettsäuren bei Kultivierung in Gegenwart von 130 µM ALA.
**Fig. 5** zeigt den Gehalt verschiedener Fettsäuren bei Kultivierung ohne ALA mit anschließendem Temperaturwechsel ("Kaltstellung").
**Fig. 6** zeigt den prozentualen Gehalt verschiedener Fettsäuren bei Kultivierung in Gegenwart verschiedener Konzentrationen von ALA und Temperaturwechsel; **6a:** Zellkultur mit 32,5 µM ALA; 6b: Zellkultur mit 65 µM ALA; **6c:** Zellkultur mit 130 µM ALA.

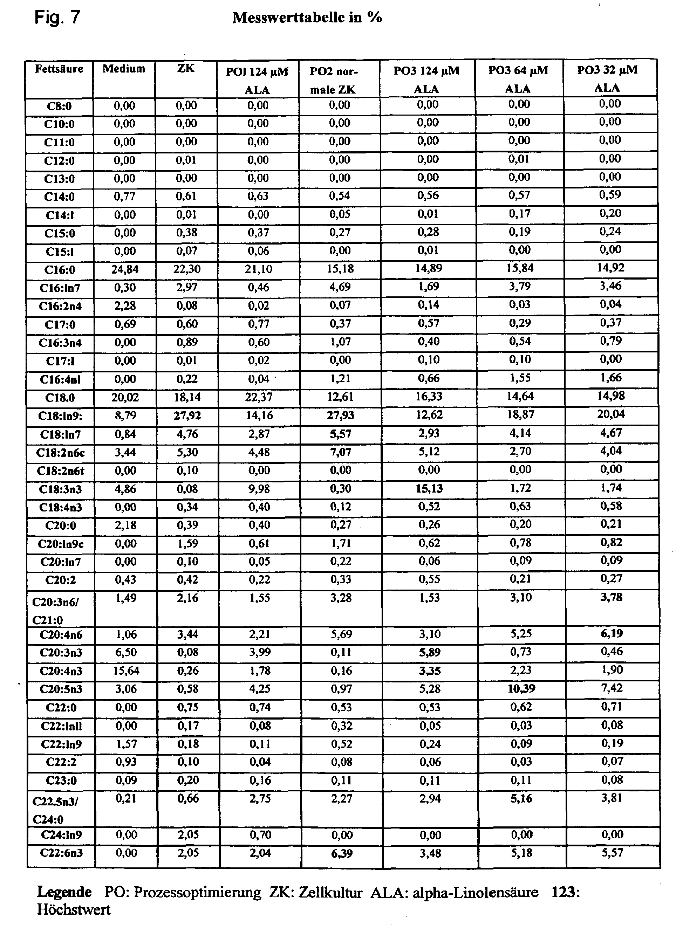

**Fig. 7** zeigt die Messwerttabelle (%) für die einzelnen Fettsäuren im Ausgangsnährmedium und in den Fischzellen, die unter den Bedingungen der Referenzbeispiele 1-3 und des Beispiels erhalten wurden.

Die folgenden Beispiele sollen die Erfindung näher erläutern ohne diese jedoch darauf zu beschränken.

### REFERENZBEISPIEL 1

### In vitro-Kultivierung von Zellen des Atlantischen Störs (Acipenser oxyrinchus) bei 25 °C und deren Charakterisierung

### 1. Etablierung einer proliferierenden Zelllinie aus dem Gewebe einer Fischlarve von A. oxyrinchus

### Isolationsprotokoll der AOXlar7-Fischzellkultur:

Verwendet wurden A. oxyrinchus-Larven im Dottersackstadium. Jede der zu präparierenden Larven wurde vor der Präparation in einem Becherglas, gefüllt mit 20% MS222 (Sigma) in Aqua dest., gegeben um diese zu betäuben. Nach 5 Minuten wurden die Larven aus dem Betäubungsbad entnommen und je eine Larve in ein 1,5-ml-Reaktionsgefäß, das mit 1 ml Trypsin (PAA) gefüllt war, überführt. Die darin enthaltene Larve wurde mit einer Schere in sehr kleine Stücke geschnitten. Der Zerkleinerungsvorgang dauerte mindestens 1 und höchstens 2 min. Im Anschluss wurden die Gewebestücke samt Trypsin mit einer geeigneten Pipette in ein 15-ml-Reaktionsgefäß mit 5 ml DMEM, das mit 20% FKS versetzt war, gegeben, um die Trypsin-Reaktion zu beenden. Die Larve in dem 15-ml-Reaktionsgefäß wurde 5 min bei 130 rcf und Raumtemperatur zentrifugiert. Nach der Zentrifugation wurde der Überstand abgesaugt und das Zellpellet mit frischem 20%i-gem DMEM aufgenommen und in eine Zellkulturschale überführt. Die so entstandene Primärzellkultur wurde zusätzlich mit 100 µg/ml Gentamycin und 2,5 µg/ml Amphotericin versetzt. Die Zellkultur wurde bei 20°C und 2,0 % CO₂ gehalten. Der erste Medienwechsel erfolgte nach 1-2 Tagen, danach wurde zweimal wöchentlich das Kulturmedium gewechselt. Nach Erreichen der Konfluenz wurde die Zellkultur schrittweise auf größere Zellkulturgefäße überführt bzw. auf mehrere Kulturgefäße aufgeteilt (Protokoll für den Trypsin-Verdau siehe 2. **Zellkultur und weitere Behandlung der Zellen).** Nachdem eine gut proliferierende Kultur angelegt worden war, wurde der Gehalt an FKS im Medium von 20% auf 10 % reduziert.

### 2. Zellkultur und weitere Behandlung der Zellen

Verwendet wurde eine AOXlar7-Fischzellkultur einer Larve des Atlantischen Störs, die wie oben unter Abschnitt 1 beschrieben erhalten worden war. Für die Versuchsreihen wurde diese aus dem Cryostock der Fraunhofer EMB aufgetaut. Während des Versuchs befanden sich die hochproliferativen Zellen zwischen den Passagen 12 und 46. Das verwendete Zellkulturmedium wurde jeweils frisch hergestellt und entsprach folgender protokollgemäßer Zusammensetzung: 500 ml DMEM, welches 10% FKS, 100 U/ml Penicillin und 0,1 mg/ml Streptomycin enthielt. Statt diesem Medium könnte jedoch auch ein anderes übliches Zellkulturmedium, vorzugsweise ein für die Kultivierung von Fischzellen bekanntes Medium verwendet werden. Insbesondere kann statt FKS (fetalem Kälberserum) auch Karpfenserum oder ein anderer äquivalenter Zusatz verwendet werden.

Die Zellen wurden in T150 Zellkulturflaschen der Firma TPP kultiviert und expandiert. Die für eine Analyse benötigte Menge an Zellen wurde vorab in einer Probemessung bestimmt und auf die Anzahl der Zellkulturflaschen umgerechnet. Die optimale Wachstumstemperatur für Störzellen wurde von S. Noglick etabliert [Noglick, Diplomarbeit, Universität Lübeck (2009)]. Sie beträgt 25°C. Für jedes Replikat bei 25°C wurden 8 T150 Zellkulturflaschen verwendet. Das Volumen des Zellkulturmediums betrug abweichend vom üblichen EMB-Protokoll 25 ml statt 30 ml. Hierfür wurden Wachstumsstudien im Vorfeld aufgenommen, in denen gezeigt werden konnte, dass ein reduziertes Medienvolumen sich positiv auf die Zellexpansion auswirkt. Die Startzellzahl je Zellkulturflasche betrug ungefähr 3 x 10⁶ Zellen, die Zellen wurden bei Erreichen der Konfluenz passagiert oder geerntet. Alle gewonnenen Proben sind im *ttz-Bremerhaven* in der Abteilung Analytik qualitativ und quantitativ auf ihr Fettsäurespektrum hin untersucht worden. Die dargestellten Ergebnisse sind Mittelwerte von Dreifachbestimmungen. Die genaue technische Durchführung der qualitativen und quantitativen Analyse wird im nächsten Abschnitt beschrieben.

Die Inkubation *der in vitro* kultivierten Zellen wurde in speziellen Zellkulturinkubatoren (Binder KBF 115) durchgeführt. Die Kultivierungsbedingungen wurden über den gesamten Versuchszeitraum mit 25°C/ 2,2% CO₂ in der Inkubatorluft und 70% Luftfeuchte konstant gehalten. Die Zellen wurden bei Erreichen der Konfluenz nach etwa 2-4 Tagen streng nach Protokoll passagiert oder geerntet. Hierzu wurde das Kulturmedium abgesaugt und die Zellen mit 1x PBS (Gibco) gespült. Danach wurden die Zellen mit genügend Trypsin-EDTA (PAA) bedeckt und 1 min bei 37°C inkubiert. Anschließend wurden die Zellen mit einem Zellschaber von der Plastikoberfläche entfernt. Der Trypsin-Verdau wurde durch die Zugabe von mindestens dem doppelten Volumen Kulturmedium gestoppt. Die Zellsuspension wurde in ein 50 ml Reaktionsgefäß überführt und zentrifugiert. Die Zentrifugationsbedingungen bei diesem Protokoll sind 130 rcf für 5 min. Das entstandene Zellpellet in dem 50 ml Reaktionsgefäß (Falcon) wurde entweder genutzt, um die Zellen in neue Zellkulturflaschen auszusäen oder in einem Gefriertrocknungsapparat (Zirbus VaCo 2-11-E) abhängig von der Pelletgröße 5-8 Stunden zu trocknen. Das Zellpellet wurde vor der Gefriertrocknung einmal mit 1 x PBS gespült und erneut bei 130 rcf 5 min zentrifugiert und dadurch von anhaftendem Zellkulturmedium befreit. Nach erfolgreicher Gefriertrocknung wurde das Zellpellet mit dem inerten Gas N₂ überschichtet, um Oxidationen der Fettsäuren durch Luftsauerstoff zu vermindern, und bei -80°C gelagert. Für den Versand der gefriergetrockneten Zellen wurden diese aus dem Reaktionsgefäß entnommen und in ein Braunglasfläschchen überführt und gewogen. Die getrockneten Fischzellen wurden erneut mit N₂-Gas überschichtet und das Fläschchen mit einem Deckel mit Septum verschlossen, um per Post zum Analyseinstitut verschickt werden zu können.

### 3. Beschreibung der Messmethode

Die Fettsäureester wurden nach Zugabe eines internen Standards (C19:0) mit einem Dichlormethan/Methanol-Gemisch im Ultraschallbad extrahiert. Nach mehreren Waschvorgängen wurde das Veresterungsreagenz (Schwefelsäure/Methanol) zugesetzt und nach erfolgter Umsetzung die gebildeten Fettsäuremethylester mit Hexan extrahiert. Dieser Extrakt wurde in einen Gaschromatographen mit selektiver Kapillarsäule (DB-FFAP) injiziert und die Fettsäuremethylester wurden nach erfolgter Trennung mit einem Flammenionisationsdetektor (FID) detektiert. Mit diesem Verfahren konnten 40 Fettsäuremethylester detektiert und über den zugesetzten internen Standard quantifiziert werden. In der nachfolgenden Tabelle sind die entsprechenden Fettsäureester aufgeführt.

**Tabelle 1**

| | | |
|---|---|---|
| 1 | C8:0 | |
| 2 | C10:0 | |
| 3 | C11:0 | |
| 4 | C12:0 | |
| 5 | C13:0 | |
| 6 | C14:0 | |
| 7 | C14:1 | |
| 8 | C15:0 | |
| 9 | C15:1 | |
| 10 | C16:0 | |
| 11 | C16 :1n7 | |
| 12 | B C16:2n4 | |
| 13 | C17:0 | |
| 14 | B_C16:3n4 | |
| 15 | C17:1 | |
| 16 | B C16:4n1 | |
| 17 | C18.0 | |
| 18 | C18:1n9:c/t | |
| 19 | B C18:1n7 | |
| 20 | C18:2n6c | |
| 21 | C18:2n6t | |
| 22 | **C18:3n3** | **ALA** |
| 23 | B C18:4n3 | |
| 24 | C20:0 | |
| 25 | C20:1n9c | |
| 26 | B C20:1n7 | |
| 27 | C20:2 | |
| 28 | C20:3n6/C21:0 | |
| 29 | C20:4n6 | |
| 30 | C20:3n3 | |
| 31 | B C20:4n3 | |
| 32 | **C20:5n3** | **EPA** |
| 33 | C22:0 | |
| 34 | B C22:1n11 | |
| 35 | C22:1n9 | |
| 36 | C22:2 | |
| 37 | C23:0 | |
| 38 | B C22.5n3 | |
| 39 | C24:1n9 | |
| 40 | **C22:6n3** | **DHA** |

Die Nachweisgrenzen für die einzelnen Komponenten lagen im Bereich um 20 µg/g Substanz.

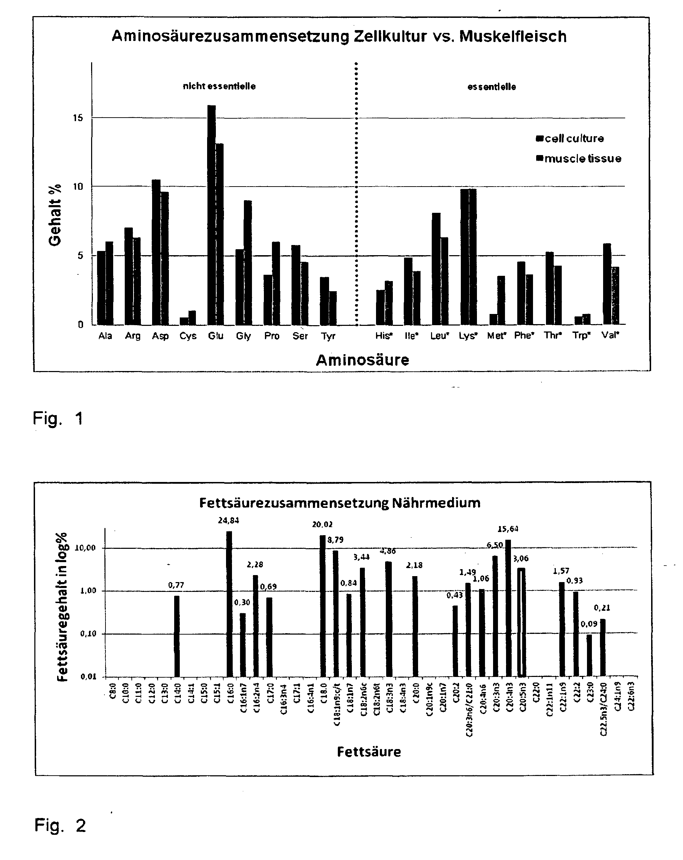

In den Zellen der *in vitro*-Zellkultur konnten Fettsäuren nachgewiesen werden, die nicht additiv über das Nährmedium zugefügt worden waren (vgl. Fig. 2 "Fettsäurezusammensetzung Nährmedium" und Fig. 3 "normale Zellkultur"). Es sind 13 Fettsäuren vertreten, die das Zellkulturmedium nicht enthält. Daraus ergibt sich die Schlussfolgerung, dass die Zellen in der Lage sind, diese Fettsäuren in vitro selbst zu synthetisieren. Auffällig ist, dass gerade die langkettigen Fettsäuren und die mehrfach ungesättigten Fettsäuren mit einem großen Anteil in den Zellen der Zellkultur zu finden sind.

In einem anderen Versuch wurde die Aminosäurezusammensetzung untersucht. Hierbei wurde das Muskelfleisch des Atlantischen Störs qualitativ und quantitativ auf den Aminosäuregehalt getestet und mit der *in vitro*-Zellkultur verglichen (vgl. Fig. 1 "Aminosäurezusammensetzung Zellkultur vs. Muskelfleisch"). Zusammenfassend lässt sich feststellen, dass die *in vitro-*Zellkultur einen ähnlichen Gehalt an Aminosäuren wie nativer Muskel des Fisches aufweist. Bezüglich 10 der getesteten 18 Aminosäuren ist in der *in vitro*-Fischzellkultur ein höherer Gehalt an Aminosäuren festzustellen.

### REFERENZBEISPIEL 2

### In vitro-Kultivierung von Zellen des Atlantischen Störs bei 25 °C in Gegenwart von ALA und deren Charakterisierung

Zur Erhöhung des Fettsäuregehalts in der Fischzellenkultur des obigen Referenzbeispiels wurde dem Zellkulturmedium aus dem Referenzbeispiel 1 eine kurzkettige dreifach ungesättigte Fettsäure, alpha-Linolensäure (ALA, 18:3n3) (Sigma, linolenic acid >99%), zugesetzt. ALA ist die Ursprungsfettsaure in der *in vivo*-Synthese der langkettigen mehrfach ungesättigten Omega-3-Fettsäuren [Das, Biotechnol. J., 1(4):420-439) (2006), Tocher et al., Proceedings of the 26th Annual Larval Fish Conference, 46 :211-2227) (2003)]. Um diese Fettsäure in das Zellkulturmedium zu inkorporieren, wurde ein Prozess gesucht, der ohne Lösungsmittel auskommt. Dieser Prozess wurde wie folgt vollzogen:
In einem 15 ml Reaktionsgefäß wurden 5 ml Zellkulturmedium vorgelegt. Zu diesen 5 ml wurde 1 µl der kurzkettigen Fettsäure mit einer 0, 5 - 10 µl Eppendorfpipette hinzugefügt. Im direkten Anschluss daran wurde das Reaktionsgefäß in einem Ultraschallgerät (Elma Elmasonic S 30 H) 5 min bei Zimmertemperatur mit Ultraschallwellen beschallt. Aus theoretischen Überlegungen heraus ergeben sich zwei verschiedene Möglichkeiten, wie die Fettsäure in Lösung geht.

Diese Fettsäure ist unlöslich in Wasser, da sie neben einer kleinen hydrophilen Carbonsäuregruppe einen langen hydrophoben Kohlenstoffkettenanteil besitzt. In erster Linie entstehen also mizellare Strukturen der Fettsäure in der Flüssigkeit. Diese Mizellen entstehen durch den schmalen Gefäßinnendurchmesser des Falconröhrchens. Die Fettsäuremoleküle lagern sich aufgrund ihrer geringen Löslichkeit in Wasser an der Phasengrenze flüssig-zu-gasförmig ein. Da nicht alle Fettsäuremoleküle an der Phasengrenze Platz finden, überschreitet die Fettsäure ihre Mizellbildungskonstante (CMC) und es bilden sich spontan Mizellen aus. Durch den zusätzlichen Energieeintrag von Ultraschallwellen in das System wird die Mizellbildung wahrscheinlich gefördert, die Größe der Mizellen reduziert und eine homogenere Verteilung erreicht.

Ein zweiter Mechanismus beruht auf einer Wechselwirkung mit Komponenten des Zellkulturmediums. Das Zellkulturmedium enthält neben den Salzen und Aminosäuren des DMEM auch 10% fötales Kälberserum (oder optional ein anderes Serum). Die in einem solchen Serum enthaltenen Proteine, besonders die Lipoproteine, sind theoretisch in der Lage, einen Anteil der Fettsäure zu binden und in der Lösung zu halten.

Zuerst wurde Zellkulturmedium ohne Zusatz und dann das mit ALA angereicherte Medium nacheinander zu der Zellkultur gegeben, um ein Endvolumen von 25 ml zu erhalten. Die Kultivierungsbedingungen entsprachen denen der Zellkultur des Referenzbeispiels. Die einzige Abweichung besteht darin, dass sich das Zellkulturmedium aus folgenden Komponenten zusammensetzt: 20 ml normales Zellkulturmedium und 5 ml normales Zellkulturmedium angereichert mit 1 µl ALA nach oben genanntem Protokoll. Die Kulturbedingungen, das Ernten der Zellen und die Lagerung entsprachen dem im Referenzbeispiel oben beschriebenen Protokoll.

Berechnung des Fettsäuregehalts von ALA bei maximaler Verteilung im Zellkulturmedium:
Dichte: 0,914 g/ml
Molmasse: 278,43 g/mol

Unter der Voraussetzung, dass die Fettsäure vollständig in der Flüssigkeit gelöst wurde, kann man die Konzentration der Fettsäure in µM (µmol/1) berechnen:
1 µl Fettsäure entspricht 0,000914 g.
0,000914 g Fettsäure entsprechen 3,28 x 10⁻⁶ mol (3,28 µmol).
3,28 µmol verteilt in 0,025 1 l Zellkulturmedium entspricht 130 µmol/l (130 µM).

Durch die Anreicherung des Zellkulturmediums mit 130 µM ALA auf die oben beschriebene Weise und anschließende Kultivierung wie im Referenzbeispiel 1 beschrieben (im Folgenden kurz als Prozessoptimierung 1 bezeichnet) konnte ein direkter Effekt beobachtet werden. Der Gehalt an ALA [schwarzer Balken in den Figuren 4-6] in den *in vitro* kultivierten Fischzellen war um den Faktor 125 (vgl. normale Zellkultur [Fig. 3; 0,08%] und Zellkultur mit 130 µM ALA [Fig. 4; 9,98%]) erhöht. Neben der Zunahme der ALA konnte auch gezeigt werden, dass die Gesamtheit der Fettsäuren, die eine längere Kohlenstoffkette als ALA tragen, also rechts vom entsprechende Balken auftauchen, erheblich zunahm (vgl. normale Zellkultur [Fig. 3; Summe = 15,52%] und Zellkultur mit 130 µM ALA [Fig. 4; Summe = 22,08%]). Dies belegt, dass die Fischzellen in der in vitro-Fischzellkultur in der Lage sind, den Zusatz an kurzkettigen Fettsäuren in langkettige, höherwertige Fettsäuren zu verstoffwechseln.

### REFERENZBEISPIEL 3

### In vitro-Kultivierung von Zellen des Atlantischen Störs mit Temperaturwechsel

Die Zellen wurden in 10 T150 Zellkulturflaschen mit der Zellzahl von annähernd 3 x 10⁶ Zellen eingesät. Es wurde davon ausgegangen, dass bei geringeren Temperaturen kein Wachstum bis zur Konfluenz in derselben Zeit wie bei 25°C erreicht werden würde. Um dennoch eine ausreichende Zellzahl für eine qualitative und quantitative Analyse zu haben, wurden statt 8 gleich 10 Zellkulturflaschen kultiviert. Die Zellen wurden 48 Stunden bei 25 °C kultiviert, um die Zellzahl zu expandieren und eine annähernde Konfluenz zu erreichen. Nach 48 Stunden wurde ein Medienwechsel vorgenommen und die Temperatur um 5 °C auf 20 °C erniedrigt. Die Kultivierungszeit bei 20 °C betrug weitere 48 Stunden. Im direkten Anschluss wurde die Temperatur auf 15 °C reduziert und die Zellen 72 Stunden bei dieser Temperatur kultiviert. Nach diesen 72 Stunden wurden die Zellen nach dem Protokoll des Referenzbeispiels 1 geerntet und die Zellzahl bestimmt. Im Anschluss wurden die Zellen ebenfalls analog zum Protokoll des Referenzbeispiels 1 (das Referenzbeispiel 1 wird in den Figuren kurz "Zellkultur" genannt) erst lyophilisiert und dann gelagert.

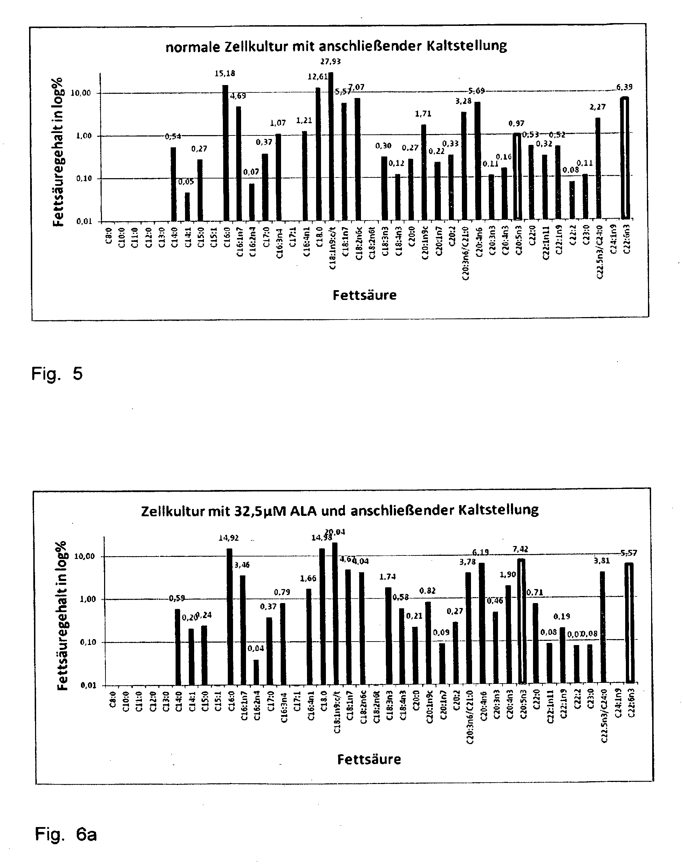

Bei Veränderung der Kulturtemperatur der Fischzellen konnte ebenfalls eine Anreicherung der Fettsäuren festgestellt werden. Die Gesamtheit der längeren Fettsäuren als ALA, also rechts vom entsprechenden Balken, nahm erheblich zu (vgl. normale Zellkultur [Fig. 3; 15,52%] und normale Zellkultur mit anschließender Kaltstellung [Fig. 5; 23,08%]). Besonders der Gehalt an EPA und DHA nahm zu. Der Gehalt an EPA erhöhte sich um den Faktor 1,6, der an DHA um den Faktor 3,1.

Durch die zeitweilige Kultivierung bei tiefer Temperatur (in den Figuren kurz als "Kaltstellung" bezeichnet) wird mutmaßlich die verstärkte Einlagerung von HUFAs in die Membran der Fischzellen (zur Erhöhung der Membranfluidität bei niedrigen Temperaturen) induziert.

### BEISPIEL

### In vitro-Kultivierung von Zellen des Atlantischen Störs mit Temperaturwechsel und in Gegenwart von ALA

Untersuchungen der Erfinder ergaben, dass die besten Ergebnisse erzielt werden, wenn der Zusatz einer kurzkettigen Fettsäure gemäß Referenzbeispiel 2 (im Folgenden und in den Figuren als "Prozessoptimierung 1" bezeichnet), und der Temperaturwechsel gemäß Referenzbeispiel 3 (im Folgenden und in den Figuren als "Prozessoptimierung 2" bezeichnet) kombiniert werden ("Prozessoptimierung 3").

Dafür wurden die Zellen, wie in Prozessoptimierung 1, mit zusätzlicher ALA versetzt und, wie in Prozessoptimierung 2, nach 48 Stunden bei 25°C, weitere 48 Stunden auf 20°C temperiert und im Anschluss daran 72 Stunden auf 15°C temperiert und geerntet. Untersucht wurden vier verschiedene Konzentrationen an zugesetzter ALA nach dem Protokoll der Prozessoptimierung 1, 0 µM, 32,5 µM, 65 µM und 130 µM ALA.

Die Zugabe von 130 µM ALA entspricht der Prozessoptimierung 1 mit anschließendem Kühlungsprozess aus Prozessoptimierung 2.

Die Zugabe von 65 µM ALA wurde wie folgt vorgenommen. Die ausgesäte Zellzahl entsprach in etwa 3 x 10⁶ Zellen je Zellkulturflasche. Die ALA wurde ebenfalls wie in Prozessoptimierung 1 in 5 ml Zellkulturmedium gelöst und in zwei Anteilen mit je 2,5 ml in zwei Zellkulturflaschen aufgeteilt. Das Medium in den Zellkulturflaschen setzte sich also aus 22,5 ml Zellkulturmedium und 2,5 ml mit ALA angereichertem Zellkulturmedium zusammen. Die Endkonzentration entsprach idealer Weise 0,5 µl ALA in 25 ml, was 65 µM ALA entspricht.

Die Zugabe von 32,5 µM ALA wurde analog der Zugabe von 65 µM vorgenommen. Die Menge an Medium wurde allerdings nicht auf zwei Zellkulturflaschen verteilt, sondern auf vier und das Gesamtmedium wurde ebenfalls angepasst. In diesem Fall beträgt die ALA-Konzentration idealerweise 0,25 µl in 25 ml und somit 32,5 µM.

Die Zugabe von 0 µM ALA entspricht dem Ergebnis der Prozessoptimierung 2.

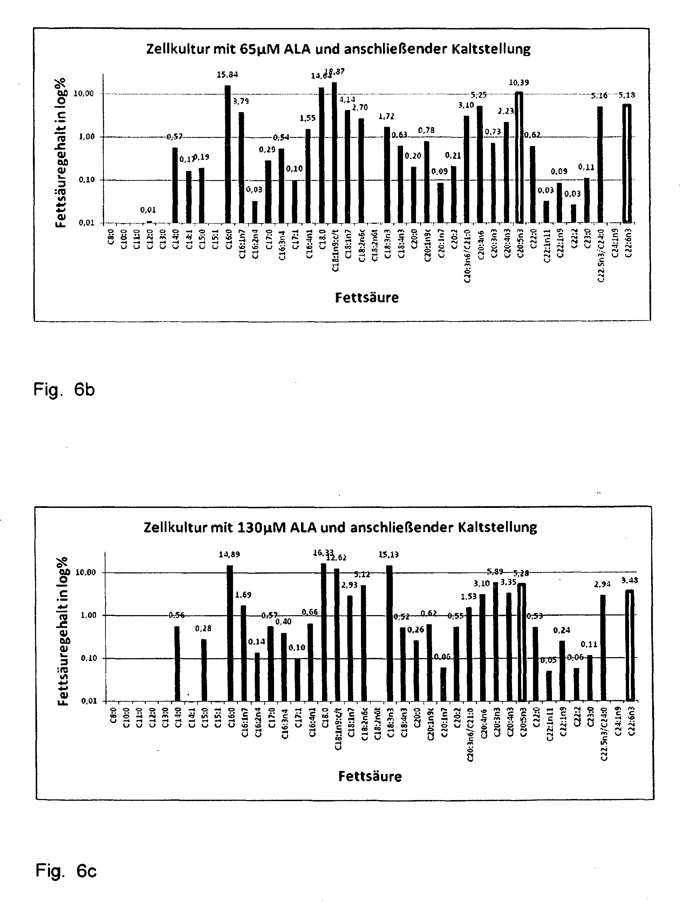

Durch die Kombination der Prozessoptimierungen 1 und 2 konnten folgende Ergebnisse erzielt werden. Die Gesamtheit der Fettsäuren, die eine längere Kohlenstoffkette als ALA tragen nahm um ca. 13, 19 und 16,5% zu (vgl. Fig. 3 "normale Zellkultur" [15,52%], Fig. 6c "Zellkultur mit 130 µM ALA mit anschließender Kaltstellung" [28,57% entspricht +13%], Fig. 6b "Zellkultur mit 65 µM ALA mit anschließender Kaltstellung" [34,83% entspricht +19%] und Fig. 6a "Zellkultur mit 32,5 µM ALA mit anschließender Kaltstellung" [32,23% entspricht +16,5%]). Hier zeigt sich, dass gerade bei dem Zusatz von 65 µM ALA die Höchstwerte an EPA [10,4%] und DHA [5,2%] erreicht werden. Das entspricht einem EPA:DHA Verhältnis von 2:1 und einem Gesamtgehalt von über 15% EPA und DHA wenn 65 µM ALA zugesetzt werden und die Zellen im Anschluss wie oben beschrieben bei kälteren Temperaturen kultiviert worden sind. Es konnte darüber hinaus eine Zunahme langkettiger Fettsäuren von über 19 % nachgewiesen werden. Die Prozessoptimierung 3 führte zu einer Reduktion der eingesetzten ALA um 50 % von 130 µM auf 65 µM. Bei dieser Reduktion hatten die Gehalte von EPA und DHA ihre Höchstwerte. Dadurch kann der Verbrauch an ALA im Prozess minimiert werden. Insgesamt erhöhte sich der Gehalt an EPA in der Zellkultur mit 65 µM und anschließender Kaltstellung um den Faktor 18 im Vergleich zur normalen Zellkultur. Bei DHA ergibt die Erhöhung den Faktor 2,5. Zu beachten ist, dass sich der Gehalt an DHA durch alleiniges Kaltstellen der normalen Zellkultur um den Faktor 3,1 erhöhen ließ. Es ist also möglich, durch geeignete Prozesssteuerung den Gehalt an gewünschten Fettsäuren gezielt zu steuern.

## Patentansprüche

1. Verfahren zur Produktion von Fischzellen mit einem erhöhten Gehalt an hochgradig ungesättigten Fettsäuren (HUFAs), die eine Kette von 20 oder mehr Kohlenstoffatomen und drei oder mehr Kohlenstoff-Doppelbindungen aufweisen, umfassend das Kultivieren und Vermehren von proliferierenden Fischzellen bei einer Temperatur von 1-35 °C in einem Zellkulturmedium, **dadurch gekennzeichnet, dass** das Kultivieren und Vermehren der proliferierenden Fischzellen unter den folgenden Bedingungen geschieht:
a) das Zellkulturmedium enthält einen Zusatz von mindestens einer kurzkettigen, mehrfach ungesättigten Fettsäure, die aus der Gruppe aus Alpha-Linolensäure (ALA, C18:3n3), Palmitoleinsäure (C16:1n7), Ölsäure (C18:1n9) und Linolsäure (C18:2n6) ausgewählt ist, in einer Konzentration von 1-500 µM;
b) das Kultivieren der Fischzellen erfolgt bei mindestens 2 verschiedenen Temperaturen, wobei die Fischzellen zuerst bei einer hohen Temperatur im Bereich von 20-30 °C, welche etwa dem Wachstumsoptimum der Fischzellen entspricht, und dann bei einer niedrigen Temperatur unterhalb von 20 °C, die mindestens 5-15 °C tiefer als die hohe Temperatur liegt, kultiviert werden;
und wobei der Gehalt an hochgradig ungesättigten Fettsäuren wie oben definiert gegenüber dem Gehalt in Fischzellen der gleichen Fischzelllinie, die bei einer Kultivierung im gleichen Zellkulturmedium ohne einen Zusatz von mindestens einer kurzkettigen ungesättigten Fettsäure wie oben definiert und bei einer Temperatur, die dem Wachstumsoptimum dieser Fischzellen entspricht, erhalten werden, um einen Faktor von mindestens 2 erhöht ist.

2. Verfahren nach Anspruch 1, **dadurch gekennzeichnet, dass** die erhaltenen Fischzellen einen erhöhten Gehalt an Eicosapentaensäure (EPA, C20:5n3) und/oder Docosahexaensäure (DHA, C22:6n3) aufweisen.

3. Verfahren nach Anspruch 1 oder 2, **dadurch gekennzeichnet, dass** das Zellkulturmedium die kurzkettige mehrfach ungesättigte Fettsäure in einer Konzentration von 10 bis 500 µM, vorzugsweise 10-300 µM, bevorzugter 20 bis 200 µM, enthält.

4. Verfahren nach einem der Ansprüche 1-3, **dadurch gekennzeichnet, dass** die hohe Temperatur etwa dem Wachstumsoptimum der Fischzellen entspricht und die niedrige Temperatur mindestens 8-12 °C, z.B. 10-20 °C oder 10-30 °C, tiefer liegt.

5. Verfahren nach einem der Ansprüche 1 bis 4, **dadurch gekennzeichnet, dass** zwischen der Kultivierung bei der hohen Temperatur und der Kultivierung bei der niedrigen Temperatur noch eine Kultivierung der Fischzellen bei mindestens einer dazwischen liegenden Temperatur erfolgt.

6. Verfahren nach Anspruch 5, **dadurch gekennzeichnet, dass** die Fischzellen für einen Zeitraum von 1-3 Tagen, vorzugsweise 40-55 h, bei der hohen Temperatur, für einen Zeitraum von 1-3 Tagen, vorzugsweise 40-55 h, bei der mittleren Temperatur, und für einen Zeitraum von 2-4 Tagen, vorzugsweise ca. 60-80 h, bei der niedrigen Temperatur kultiviert werden.

7. Verfahren nach einem der Ansprüche 1 bis 6, **dadurch gekennzeichnet, dass** die proliferierenden Fischzellen aus einer Fischzelllinie stammen, die aus dem Gesamtgewebe einer Fischlarve oder einem speziellen Gewebe eines adulten Fisches, insbesondere aus der Niere, Kopfniere, dem Gehirn, der Leber, dem Pankreas, den Pylorusanhängen, den Gonaden, dem Darm, Herz, der Haut oder aus Muskelgewebe gewonnen wurde.

8. Verfahren nach einem der Ansprüche 1 bis 7, **dadurch gekennzeichnet, dass** die Spezies der Fischzellen aus einer Gruppe von Spezies ausgewählt ist, welche Stör, Hering, Forelle, Lachs, Aal, Karpfen, Makrele, Heilbutt, Sardine umfasst.

9. Fischzellkultur, umfassend proliferierende Fischzellen in einem Zellkulturmedium, das einen Zusatz von mindestens einer kurzkettigen ungesättigten Fettsäure enthält, die aus der Gruppe aus Alpha-Linolensäure (ALA, C18:3n3), Palmitoleinsäure (C16:1n7), Ölsäure (18:1n9) und Linolsäure (C18:2n6) ausgewählt ist, und eine Temperatur im Bereich von 1-20 °C aufweist, wobei die proliferierenden Fischzellen aus einer Fischzelllinie stammen, die aus dem Gesamtgewebe einer Fischlarve oder aus der Niere, Kopfniere, dem Gehirn, dem Pankreas, den Pylorusanhängen, den Gonaden, dem Darm, Herz, der Haut oder aus Muskelgewebe eines adulten Fisches gewonnen wurde, und die proliferierenden Fischzellen einen Gehalt an hochgradig ungesättigten Fettsäuren, insbesondere Eicosapentaensäure (EPA, C20:5n3) und/oder Docosahexaensäure (DHA, C22:6n3), aufweisen, der gegenüber dem Gehalt in Fischzellen der gleichen Fischzelllinie, die bei einer Kultivierung im gleichen Zellkulturmedium ohne einen Zusatz von mindestens einer kurzkettigen ungesättigten Fettsäure und bei einer Temperatur, die dem Wachstumsoptimum dieser Fischzellen entspricht, erhalten werden, um einen Faktor von mindestens 2, bevorzugter mindestens 3, z.B. mindestens 5, erhöht ist.

10. Fischzellkultur nach Anspruch 9, **dadurch gekennzeichnet, dass** die Spezies der Fischzellen aus einer Gruppe von Spezies ausgewählt ist, welche Stör, Hering, Forelle, Lachs, Aal, Karpfen, Makrele, Heilbutt, Sardine umfasst.

11. Verwendung der Fischzellkultur nach Anspruch 9 oder 10 zur Gewinnung von fischspezifischen Produkten, einschließlich von Fischmehl, Fischproteinen, Fischölen und Fettsäuren, insbesondere hochgradig ungesättigten Fettsäuren, welche Produkte in der Ernährungsindustrie (Nahrungsergänzungsmittel, diätetische Lebensmittel, Vitamine, Enzyme), als Tierfutterzusatz, in der Medizin oder Pharmazie eingesetzt werden können.

12. Verfahren zur Gewinnung von fischspezifischen Produkten, einschließlich von Fischmehl, Fischproteinen, Fischölen und Fettsäuren, aus Fischzellkulturen, umfassend
a) Kultivieren und Vermehren von proliferierenden Fischzellen in einem Zellkulturmedium gemäß dem Verfahren nach einem der Ansprüche 1-8,
b) Ernten der Fischzellen, und
c) Gefriertrocknen und/oder Zerkleinern und/oder Aufschließen der geernteten Fischzellen.

## Claims

1. A method for the production of fish cells with an increased content of highly unsaturated fatty acids (HUFAs), which have a chain of 20 or more carbon atoms and three or more carbon double bonds, comprising the cultivation and multiplication of proliferating fish cells at a temperature of 1-35°C in a cell culture medium, **characterized in that** the cultivation and multiplication of the proliferating fish cells take place under the following conditions:
a) the cell culture medium contains an additive of at least one short-chain polyunsaturated fatty acid, which is selected from the group consisting of alpha linolenic acid (ALA, C18:3n3), palmitolenic acid (C16:1n7), olenic acid (C18:1n9) and linoleic acid (C18:2n6), in a concentration of 1-500 µM;
b) the cultivation of the fish cell takes place at at least 2 different temperatures, wherein the fish cells are cultivated at first at a high temperature in the range of 20-30°C, which corresponds to approximately the growth optimum of the fish cells, and then at a low temperature below 20°C, which is at least 5-15°C lower than the high temperature;
and wherein the concentration of highly unsaturated fatty acids as defined above is increased with respect to the concentration in fish cells of the same fish cell line, which are obtained for a cultivation in the same cell culture medium without any additive of at least one short-chain unsaturated fatty acid as defined above and at a temperature, which corresponds to the growth optimum of these fish cells, by a factor of at least 2.

2. The method according to claim 1, **characterized in that** the fish cells obtained have an increased concentration of eicosapentaenoic acid (EPA, C20:5n3) and/or docosahexaenoic acid (DHA, C22:6n3).

3. The method according to claim 1 or 2, **characterized in that** the cell culture medium contains the short-chain polyunsaturated fatty acid in a concentration of 10 to 500 µM, preferably 10-300 µM, more preferably 20 to 200 µM.

4. The method according to any one of claims 1-3, **characterized in that** the high temperature corresponds to approximately the growth optimum of the fish cells and the low temperature is at least 8-12°C, e.g. 10-20°C or 10-30°C, lower.

5. The method according to any one of claims 1 to 4, **characterized in that**, between the cultivation at the high temperature and the cultivation at the low temperature, another cultivation of the fish cells takes place at at least one temperature which is therebetween.

6. The method according to claim 5, **characterized in that** the fish cells are cultivated for a time period of 1-3 days, preferably 40-55 h, at the high temperature, for a time period of 1-3 days, preferably 40-55 h, at the middle temperature, and for a time period of 2-4 days, preferably about 60-80 h, at the low temperature.

7. The method according to any one of claims 1 to 6, **characterized in that** the proliferating fish cells stem from a fish cell line, which was obtained from the whole tissue of a fish larva or a specific tissue of an adult fish, in particular from the kidney, head kidney, the brain, the liver, the pancreas, the pyloric caeca, the gonads, the gut, heart, the skin or from muscle tissue.

8. The method according to any one of claims 1 to 7, **characterized in that** the species of the fish cells is selected from a group of species, which comprises sturgeon, herring, trout, salmon, eel, carp, mackerel, halibut, sardine.

9. A fish cell culture, comprising proliferating fish cells in a cell culture medium, which contains an additive of at least one short-chain unsaturated fatty acid which is selected from the group consisting of alpha linolenic acid (ALA, C18:3n3), palmitolenic acid (C16:1n7), olenic acid (C18:1n9) and linoleic acid (C18:2n6) and which has a temperature in the range of 1-20°C, wherein the proliferating fish cells stem from a fish cell line, which was obtained from the whole tissue of a fish larva or from the kidney, head kidney, the brain, the pancreas, the pyloric caeca, the gonads, the gut, heart, the skin or from muscle tissue and the proliferating fish cells have a concentration of highly unsaturated fatty acids, in particular eicosapentaenoic acid (EPA, C20:5n3) and/or docosahexaenoic acid (DHA, C22:6n3), which is increased with respect to the concentration in fish cells of the same fish cell line, which are obtained for a cultivation in the same cell culture medium without any additive of at least one short-chain unsaturated fatty acid and at a temperature, which corresponds to the growth optimum of these fish cells, by a factor of at least 2, more preferably at least 3, e.g. at least 5.

10. The fish cell culture according to claim 9, **characterized in that** the species of the fish cells is selected from a group of species, which comprises sturgeon, herring, trout, salmon, eel, carp, mackerel, halibut, sardine.

11. Use of the fish cell culture according to claim 9 or 10 for obtaining fish-specific products, including fish meal, fish proteins, fish oils and fatty acids, in particular highly unsaturated fatty acids, which products can be used in the food industry (food supplement, dietetic food, vitamins, enzymes), as animal feed additive, in the medicine or pharmacy sectors.

12. A method for obtaining fish-specific products, including fish meal, fish proteins, fish oils and fatty acids, from fish cell cultures, comprising
a) cultivation and multiplication of proliferating fish cells in a cell culture medium according to the method according to any one of claims 1-8,
b) harvesting the fish cells, and
c) freeze-drying and/or comminuting and/or decomposition of the harvested fish cells.

## Revendications

1. Procédé de production de cellules de poisson ayant une teneur accrue en acides gras hautement insaturés (HUFA) qui présentent une chaîne de 20 atomes de carbone ou plus et trois doubles liaisons carbone ou plus, comprenant la culture et la multiplication de cellules de poisson proliférantes, à une température de 1 à 35 °C dans un milieu de culture cellulaire, **caractérisé en ce que** la culture et la multiplication des cellules de poisson proliférantes s'effectuent dans les conditions suivantes :
a) le milieu de culture cellulaire contient un additif d'au moins un acide gras à chaîne courte, poly-insaturé qui est choisi dans le groupe comprenant l'acide alpha-linolénique (ALA, C18:3n3), l'acide palmitoléique (C16:1n7), l'acide oléique (C18:1n9) et l'acide linoléique (C18:2n6), en une concentration de 1 à 500 µm ;
b) la culture des cellules de poisson s'effectue à au moins 2 températures différentes, les cellules de poisson étant tout d'abord cultivées à une température élevée dans la plage de 20 à 30 °C qui correspond à peu près aux conditions optimales de croissance des cellules de poisson puis à une température basse inférieure à 20 °C qui est au moins inférieure de 5 à 15 °C à la température élevée ;
et la teneur en acides gras hautement insaturés tels définis ci-dessus étant accrue d'un facteur d'au moins 2 par rapport à la teneur en cellules de poisson de la même lignée de cellules de poisson, qui sont obtenues dans une culture dans le même milieu de culture cellulaire sans addition d'au moins un acide gras insaturé à chaîne courte tel que défini ci-dessus et à une température qui correspond aux conditions optimales de croissance de ces cellules de poisson.

2. Procédé selon la revendication 1, **caractérisé en ce que** les cellules de poisson obtenues présentent une teneur accrue en acide eicosapentaénique (EPA, C20:5n3) et/ou en acide docosahexaénique (DHA, C22:6n3).

3. Procédé selon la revendication 1 ou 2, **caractérisé en ce que** le milieu de culture cellulaire contient l'acide gras à chaîne courte poly-insaturé en une concentration de 10 à 500 µm, de préférence de 10 à 300 µm, de manière davantage préférée de 20 à 200 µm.

4. Procédé selon l'une des revendications 1 à 3, **caractérisé en ce que** la température élevée correspond à peu près aux conditions optimales de croissance des cellules de poisson et la température basse est au moins inférieure de 8 à 12 °C, par exemple inférieure de 10 à 20 °C ou de 10 à 30 °C.

5. Procédé selon l'une des revendications 1 à 4, **caractérisé en ce qu'**entre la culture à la température élevée et la culture à la température basse s'effectue encore une culture des cellules de poisson à au moins une température se trouvant entre celles-ci.

6. Procédé selon la revendication 5, **caractérisé en ce que** les cellules de poisson sont cultivées pendant une durée de 1 à 3 jours, de préférence de 40 à 55 h, à la température élevée, pendant une durée de 1 à 3 jours, de préférence de 40 à 55 h, à la température moyenne, et pendant une durée de 2 à 4 jours, de préférence d'environ 60 à 80 h, à la température basse.

7. Procédé selon l'une des revendications 1 à 6, **caractérisé en ce que** les cellules de poissons proliférantes proviennent d'une lignée de cellules de poisson qui ont été obtenues à partir du tissu complet d'une larve de poisson ou d'un tissu spécial d'un poisson adulte, en particulier des reins, des reins antérieurs, du cerveau, du foie, du pancréas, du caecum pylorique, des gonades, de l'intestin, du coeur, de la peau ou des tissus musculaires.

8. Procédé selon l'une des revendications 1 à 7, **caractérisé en ce que** l'espèce de cellules de poisson est choisie dans un groupe d'espèces qui comprend l'esturgeon, le hareng, la truite, le saumon, l'anguille, la carpe, le maquereau, le flétan, la sardine.

9. Culture de cellules de poisson, comprenant des cellules de poisson proliférantes dans un milieu de culture cellulaire qui contient une addition d'au moins un acide gras insaturé à chaîne courte qui est choisi dans le groupe comprenant l'acide alpha-linolénique (ALA, C18:3n3), l'acide palmitoléique (C16:1n7), l'acide oléique (C18:1n9) et l'acide linoléique (C18:2n6), et une température dans une plage de 1 à 20 °C, les cellules de poisson proliférantes provenant d'une lignée de cellules de poisson qui ont été obtenues à partir du tissu complet d'une larve de poisson ou des reins, des reins antérieurs, du cerveau, du pancréas, du caecum pylorique, des gonades, de l'intestin, du coeur, de la peau ou des tissus musculaires d'un poisson adulte et les cellules de poisson proliférantes ayant une teneur en acides gras hautement insaturés, en particulier en acide eicosapentaénique (EPA, C20:5n3) et/ou en acide docosahexaénique (DHA, C22:6n3) qui est accrue par rapport à la teneur en cellules de poisson de la même lignée de cellules de poisson, qui sont obtenues dans une culture dans le même milieu de culture cellulaire sans addition d'au moins un acide gras insaturé à chaîne courte et à une température qui correspond aux conditions optimales de croissance de ces cellules de poisson, d'un facteur d'au moins 2, de préférence d'au moins 3, par exemple d'au moins 5.

10. Culture de cellules de poisson selon la revendication 9, **caractérisée en ce que** l'espèce de cellules de poisson est choisie dans un groupe d'espèces qui comprend l'esturgeon, le hareng, la truite, le saumon, l'anguille, la carpe, le maquereau, le flétan, la sardine.

11. Utilisation de la culture de cellules de poisson selon la revendication 9 ou 10, pour obtenir des produits spécifiques de poisson, comprenant de la farine de poisson, des protéines de poisson, des huiles de poisson et des acides gras, en particulier des acides gras hautement insaturés, lesdits produits pouvant être utilisés dans l'industrie alimentaire (compléments alimentaires, aliments diététiques, vitamines, enzymes), comme additifs d'aliments pour animaux, en médecine ou en pharmacie.

12. Procédé d'obtention de produits spécifiques de poisson comprenant de la farine de poisson, des protéines de poisson, des huiles de poisson et des acides gras à partir de cultures de cellules de poisson, comprenant
a) la culture et la multiplication de cellules de poisson proliférantes dans un milieu de culture cellulaire selon le procédé selon l'une des revendications 1 à 8,
b) la récolte des cellules de poisson, et
c) la lyophilisation et/ou la réduction et/ou la décomposition des cellules de poisson récoltées.
